(19) Europäisches Patentamt / European Patent Office / Office européen des brevets

(11) EP 3 881 985 A1

(12) **EUROPEAN PATENT APPLICATION**
published in accordance with Art. 153(4) EPC

(43) Date of publication:
**22.09.2021 Bulletin 2021/38**

(21) Application number: **19884817.8**

(22) Date of filing: **14.11.2019**

(51) Int Cl.:
**B25J 11/00** (2006.01)    **B25J 13/08** (2006.01)
**A61F 2/74** (2006.01)    **A61B 5/107** (2006.01)
**A61B 5/11** (2006.01)

(86) International application number:
**PCT/JP2019/044639**

(87) International publication number:
**WO 2020/100963 (22.05.2020 Gazette 2020/21)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **16.11.2018   JP 2018215536**

(71) Applicant: **BRIDGESTONE CORPORATION**
**Chuo-ku**
**Tokyo 104-8340 (JP)**

(72) Inventor: **TSUCHIDA, Shinya**
**Tokyo 104-8340 (JP)**

(74) Representative: **Oxley, Robin John George**
**Marks & Clerk LLP**
**15 Fetter Lane**
**London EC4A 1BW (GB)**

(54)    **HUMAN WEARABLE POWER ASSIST DEVICE**

(57)    A body mounting type power assist device (10) is provided with an posture detection unit (110) for detecting that a human body is in a predetermined posture, and a control unit (120) for operating a rubber actuator (140) when the posture detection unit (110) detects that the human body is in the predetermined posture.

FIG. 3

EP 3 881 985 A1

**Description**

[Technical Field]

The present invention relates to a body mounting type power assist device mounted on a human body.

[Background Art]

Conventionally, there has been known a so-called power assist suit, which is a power assist device mounted on a human body and assisting the lifting operation of a heavy object.

In such a power assist suit, a method of controlling the start of power assist by the wearer's voice has been proposed (Patent Literature 1). There has also been proposed a method of providing a load cell in a power assist suit and controlling the start of power assist based on a load applied to the load cell (Patent Literature 2).

[Citation List]

[Patent Literature]

[PTL 1] Japanese Unexamined Patent Application Publication No. 2011-251057
[PTL 2] Japanese Unexamined Patent Application Publication No. 2015-047662

[Summary of Invention]

However, the conventional power assist suit described above has the following problems. Specifically, the method of controlling the start of the power assist by the user's voice may not work well in an environment with a high noise level, such as in a factory or a construction site.

As to the method of controlling the start of the power assist based on the load applied to the load cell, although it is a short time, a burden is imposed on the wearer from the detection of a constant load by the load cell until the start of the power assist. As a countermeasure, it is conceivable to lower the threshold value of the load detected by the load cell, but it is likely to cause another problem that the power assist is easily started by mistake.

Accordingly, the present invention has been made in view of such a situation, and an object of the present invention is to provide a body-mounted power assist device capable of starting power assist at a more appropriate timing.

One aspect of the present invention is a body mounting type power assist device mounted to a human body and including an actuator assisting a predetermined operation of the human body, including a posture detection unit for detecting that the human body has reached a predetermined posture, and a control unit for actuating the actuator when the posture detection unit detects that the human body has reached the predetermined posture.

[Brief Description of Drawings]

FIG. 1 is a diagram showing a state in which a body mounting type power assist device 10 is mounted on the wearer 20.
FIG. 2A is a view showing a front view of the body mounting type power assist device 10 mounted on the wearer 20.
FIG. 2B is a view showing a side view of a body mounting type power assist device 10 mounted on a wearer 20 in a half-sitting position.
FIG. 3 is a functional block diagram of the body mounting type power assist device 10.
FIG. 4 is a side view of a rubber actuator 140.
FIG. 5 is a diagram showing a flow of an assist operation for lifting a heavy object by the body mounting type power assist device 10.

[Description of Embodiments]

Embodiments will be described below with reference to the drawings. The same functions and configurations are denoted by the same or similar reference numerals, and description thereof will be omitted as appropriate.

(1)Overall schematic configuration of body mounting type power assist device

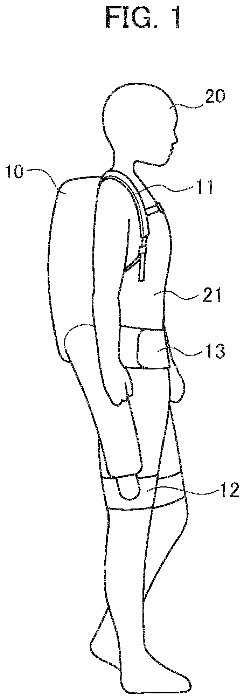

FIG. 1 shows a state in which the body mounting type power assist device 10 according to the present embodiment is mounted on the wearer 20.

As shown in FIG. 1, the body mounting type power assist device 10 is mounted on a human body 21 of the wearer 20. Specifically, the body mounting type power assist device 10 is mounted on the thigh portion and the back portion of the human body 21.

More specifically, the body mounting type power assist device 10 is mounted on the back of the human body 21 by a shoulder belt 11 and mounted on the thigh of the human body 21 by a ring-shaped support belt 12. The body mounting type power assist device 10 is mounted on the waist part of the human body 21 by a waist belt 13.

The body mounting type power assist device 10 assists the predetermined operation of the human body 21. The predetermined operation includes, for example, an operation of lifting a heavy object and an operation of holding a posture in a half sitting position.

The body mounting type power assist device 10 includes an actuator for assisting the predetermined operation. Specifically, the body mounting type power assist device 10 includes a fluid pressure actuator using a fluid such as a gas or liquid.

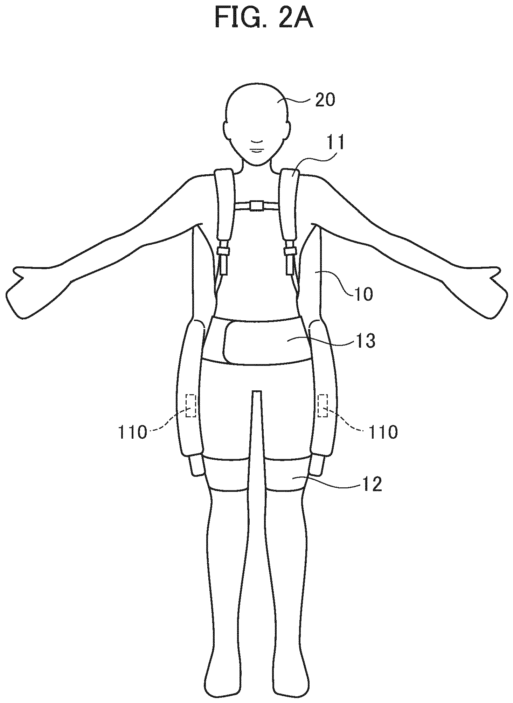

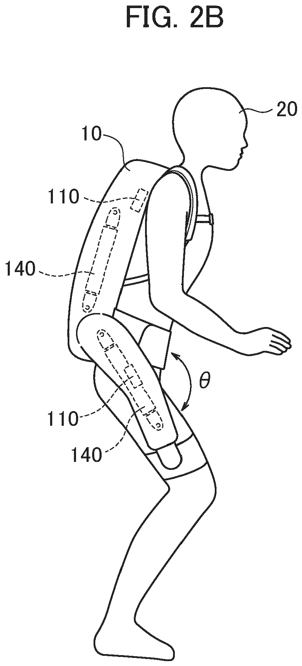

FIG. 2A shows a front view of the body mounting type power assist device 10 mounted on the wearer 20. FIG. 2B shows a side view of the body mounting type power assist device 10 mounted on the wearer 20 in a half-sitting position.

As shown in FIGS. 2A and 2B, a plurality of sensors 111, a rubber actuator 140, and the like are provided inside the body mounting type power assist device 10. Specifically, the sensor 111 is provided on the thigh portion and the back portion of the human body 21. In this embodiment, based on the acceleration and the angular velocity detected by the sensor 111, it is detected that the human body 21 is in a predetermined posture.

Specifically, the body mounting type power assist device 10 detects that the posture angle $\theta$ of the human body 21 becomes a predetermined value based on the acceleration and the angular velocity, and operates the rubber actuator 140 based on the detection result.

(2)Function block configuration of body mounting type power assist device

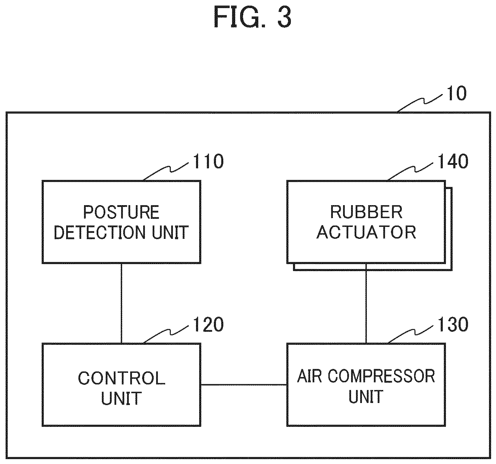

FIG. 3 is a functional block diagram of the body mounting type power assist device 10. As shown in FIG. 3, the body mounting type power assist device 10 includes a posture detection unit 110, a control unit 120, an air compressor unit 130, and a rubber actuator 140.

The posture detection unit 110 detects that the human body 21 of the wearer 20 is in a predetermined posture. Specifically, the posture detection unit 110 can detect that the wearer 20 has become in a bent posture in order to lift a heavy object. The bent posture is a state in which the wearer 20 bends his/her legs or lower back so as to lower his/her posture, which may be called a crouching position.

Alternatively, the posture detection unit 110 may detect that the wearer 20 is in a half-sitting posture. The half-sitting posture is not as low as the crouching posture, but it is a posture of lowering the posture by bending the waist and knees.

Moreover, the posture detection unit 110 can detect the timing at which the human body 21 of the wearer 20 changes from the bent posture to the rising posture as a predetermined posture.

Specifically, the posture detection unit 110 detects the timing when the waist angle of the wearer 20, specifically, the posture angle $\theta$ gradually decreases, the posture angle $\theta$ stops decreasing once, and the posture angle $\theta$ starts to increase again. By detecting such a change in the posture angle $\theta$, the timing at which the human body 21 changes from the bent posture to the rising posture can be detected.

That is, in a side view of the human body 21, the posture detection unit 110 detects that the human body 21 is in a predetermined posture based on the posture angle $\theta$ formed by the thigh portion and the back portion.

As described above, the posture detection unit 110 includes sensors 111 (see FIGs 2A and 2B) provided on the thigh portion and the back portion of the human body 21.

The sensor 111 can detect at least acceleration. The posture detection unit 110 calculates an posture angle $\theta$ based on the detected acceleration. Specifically, the posture detection unit 110 continuously detects the acceleration of the thigh portion and the back portion of the human body 21 using the sensor 111, and calculates the movement amount of the thigh portion and the back portion. The posture detection unit 110 further calculates an posture angle $\theta$ based on the movement amount.

Specifically, the sensors 111 are provided at 3 locations: the thigh portion of the left leg, the thigh portion of the right leg, and the back portion of the wearer 20. The sensor 111 may be capable of detecting not only acceleration (xyz) but also angular velocity (xyz) and geomagnetism (xyz).

The control unit 120 controls the rubber actuator 140 through the air compressor unit 130.

More specifically, when the posture detection unit 110 detects that the human body 21 of the wearer 20 is in a predetermined posture, the control unit 120 operates the rubber actuator 140. That is, when the wearer 20 assumes the predetermined posture, the body mounting type power assist device 10 (rubber actuator 140) starts assisting the operation of the wearer 20 (e.g., lifting of heavy objects).

More specifically, the control unit 120 gradually decreases the posture angle $\theta$, stops decreasing the posture angle $\theta$ once, and starts the assist at the timing when the posture angle $\theta$ starts to increase again.

The air compressor unit 130 is connected with the rubber actuator 140 and feeds compressed air used for operating the rubber actuator 140 to the rubber actuator 140. Specifically, the air compressor unit 130 includes an air compressor, a pressure tank, and a control valve (unillustrated).

The air compressor unit 130 opens and closes a control valve on the basis of a control signal from the control unit 120, and adjusts the amount of air fed to the rubber actuator 140 and the amount of air discharged from the rubber actuator 140.

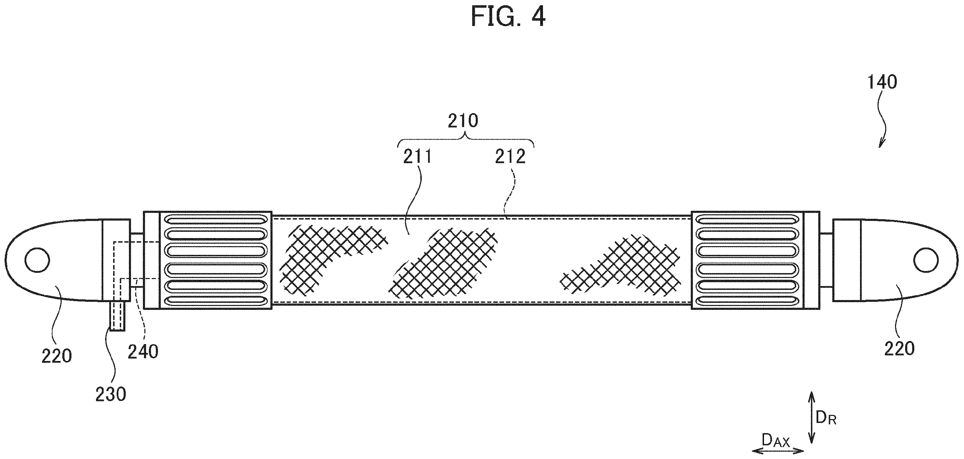

The rubber actuator 140 is a cylindrical actuator which expands and contracts by the pressure of a fluid. The rubber actuator 140 includes a cylindrical tube 211 (not shown in FIG. 3, see FIG. 4) formed of a rubber material. A specific configuration of the rubber actuator 140 will be described later.

In this embodiment, a plurality of rubber actuators 140 are used. Specifically, the body mounting type power assist device 10 includes the rubber actuator 140 provided on the back portion of the human body 21 and the rubber actuator 140 provided on the thigh portion of the human body 21.

In this embodiment, the left leg and the right leg of the wearer 20 are provided with rubber actuators 140, respectively.

The rubber actuator 140, which is connected to the rubber actuator 140 for the left leg, and provided on the back part of the human body 21, and the rubber actuator 140, which is connected to the rubber actuator 140 for the right leg, and provided on the back part of the human body 21 are used. Specifically, the body mounting type power assist device 10 includes at least one rubber actuator 140, and in this embodiment, a total of four rubber actuators.

When assisting the lifting operation of the heavy object, the rubber actuator 140 is changed from the non-pressurized state to the pressurized state at the timing when the human body 21 of the wearer 20 changes from the bent posture to the rising posture. Thus, since the rubber actuator 140 of the thigh part and the rubber actuator 140 of the back part contract, the direction in which the posture angle $\theta$ expands, that is, the rising motion can be assisted.

Further, in case of the operation of maintaining the half-sitting posture of the wearer 20 is assisted, when the posture angle $\theta$ becomes a predetermined value, the posture angle $\theta$ can be maintained at a predetermined value by operating the rubber actuator 140 of the thigh portion and the rubber actuator 140 of the back portion at an intermediate pressure (a pressure lower than that used to assist in lifting heavy objects).

(3)Configuration of fluid pressure actuator

Next, a configuration of an actuator according to the present embodiment will be described. As described above, in this embodiment, the rubber actuator 140 that is a fluid pressure actuator is used.

FIG. 4 is a side view of the rubber actuator 140 according to the present embodiment. As shown in FIG. 2, the rubber actuator 140 includes an actuator body portion 210 and a connection portion 220.

The actuator body portion 210 comprises a tube 211 and a sleeve 212. The actuator body portion 210 is extremely lightweight (about 100 g) and compact because it can be made of rubber, organic fiber, or the like, as will be described later. Fluid flows into the actuator body portion 210 through a fitting 230 and a passage hole 240. Specifically, at both ends of the rubber actuator 140, connection portion 220 are respectively provided at the end parts in the longitudinal direction of the rubber actuator 140.

The actuator body portion 210 contracts in the axial direction $D_{AX}$ of the actuator body portion 210 and expands in the radial direction $D_R$ by inflow of fluid into the tube 211. The actuator body portion 210 expands in the axial direction $D_{AX}$ of the actuator body portion 210 and contracts in the radial direction $D_R$ due to the outflow of fluid from the tube 211.

Due to the shape change of the actuator body portion 210, the rubber actuator 140 functions as an actuator. The rubber actuator 140 is a so-called McKibben type.

The fluid used for driving the rubber actuator 140 may be either a gas such as air or a liquid such as water or mineral oil, but in view of the fact that the fluid is used for the body mounting type power assist device 10, it is not necessary to perform hydraulic driving to exert a large contraction force. Therefore, in the present embodiment, as described above, air is preferably used as the fluid used for driving the rubber actuator 140. That is, the fluid flowing into the rubber actuator 140 is air.

The fitting 230 projects to attach a driving pressure source for the rubber actuator 140, specifically a hose (unillustrated) connected to a control valve (unillustrated).

The fluid flowing in through the fitting 230 passes through the passage hole 240 and flows into the inside of the actuator body portion 210, specifically into the inside of the tube 211.

The tube 211 is a cylindrical body which expands and contracts by the pressure of the fluid. The tube 211 is made of an elastic material such as butyl rubber in order to repeat contraction and expansion by fluid. That is, the tube 211 is preferably formed of a predetermined rubber material.

When the rubber actuator 140 is hydraulically driven, it is preferable to use at least one kind selected from the group consisting of NBR (nitrile rubber) having high oil resistance or hydrogenated NBR, chloroprene rubber and epichlorohydrin rubber.

The sleeve 212 is cylindrical and covers the outer peripheral surface of the tube 211. The sleeve 212 is a restraining member composed of fibers for restraining expansion of the tube 211 more than predetermined amount.

Specifically, the sleeve 212 is a stretchable structure in which cords oriented in a predetermined direction are woven, and the rhombic shape is repeated by crossing the oriented cords. By having such a shape, the sleeve 212 is pantographically deformed and follows the contraction and expansion of the tube 211 while regulating it.

As the cord forming the sleeve 212, a fiber cord of aromatic polyamide (aramid fiber) or polyethylene terephthalate (PET) is preferably used. However, the present invention is not limited to such a type of fiber cord, and may be, for example, a high-strength fiber such as a PBO fiber (polyparaphenylenebenzobisoxazole) or a metal cord made of an ultrafine filament.

The details of the rubber actuator 140 may be the same as those described in International Publication No. WO 2017/010304, for example.

(4)Operation of the body mounting type power assist device

Next, the operation of the body mounting type power assist device 10 will be described. Specifically, an operation in which the body mounting type power assist device 10 assists the wearer 20 to lift a heavy object will be described.

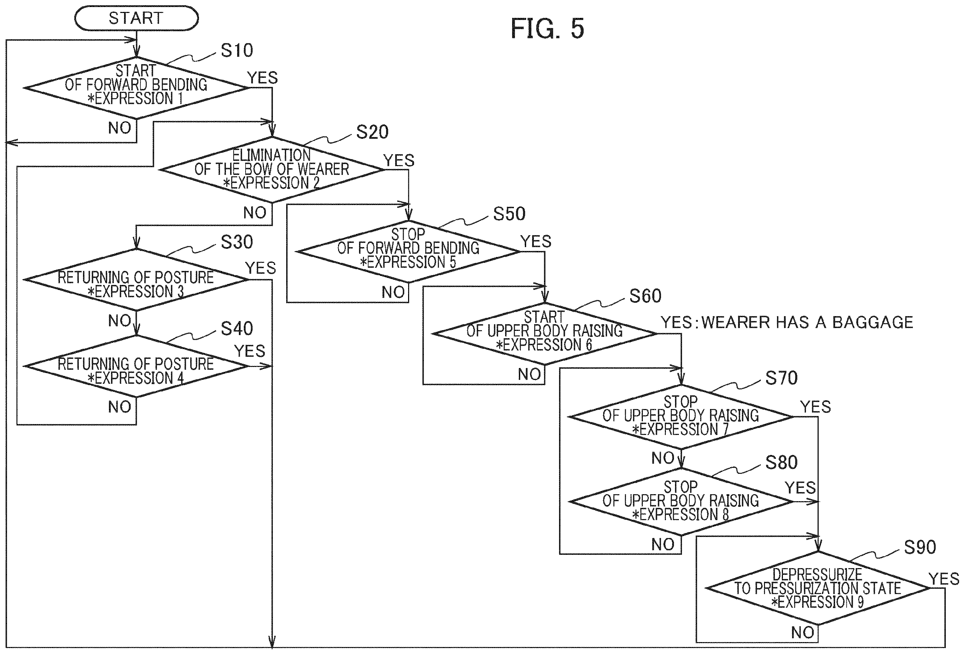

FIG. 5 shows a flow of an assist operation for lifting a heavy object by the body mounting type power assist device 10.

As shown in FIG. 5, the body mounting type power assist device 10 determines whether or not the wearer 20 has started the forward bending operation (S 10). At this point of time, it is preferable that the rubber actuator 140 be in a constant pressurized state (in the present embodiment, about 0.04 Mpa) so as to be able to respond quickly.

Specifically, the body mounting type power assist device 10 determines whether or not the wearer 20 has started the forward bending operation based on the (Expression 1).

$$\text{Left thigh acceleration} > 15\,[\text{G}] \text{ or Right thigh acceleration} > 15\,[\text{G}] \text{ or } |\text{ Left thigh acceleration - Right thigh acceleration }| > 15\,[\text{G}] \text{ and Back acceleration} > 15\,[\text{G}] \ldots \text{(Expression 1)}$$

The "Left thigh acceleration" is acceleration detected by the sensor 111 provided corresponding to the thigh portion of the left leg of the wearer 20. The "right thigh acceleration" is acceleration detected by the sensor 111 provided corresponding to the thigh portion of the right leg of the wearer 20.

The "back acceleration" is an acceleration detected by the sensor 111 provided corresponding to the back portion of the wearer 20.

The body mounting type power assist device 10 eliminates the bow of the wearer 20 from among the start of the forward bending operation determined in S 10 (S 20).

Specifically, the body mounting type power assist device 10 excludes the bowing of the wearer 20 based on the (Expression 2).

$$\text{Thigh Left Angle} > 30\,[\text{angle}] \text{ and Thigh Right Angle} > 30\,[\text{angle}] \text{ or } |\text{ Thigh Left Angle - Thigh Right Angle }| > 30\,[\text{angle}] \ldots \text{(Expression 2)}$$

The "Left Thigh Angle" is the angle formed by the thigh portion of the left leg of wearer 20 in a vertical direction. The "Thigh Right Angle" is the angle formed by the thigh portion of the right leg of the wearer 20 in a vertical direction.

If the (Expression 2) is not satisfied (NO in S 20), the body-mounted power assist device 10 determines whether or not the wearer 20 has returned from the bowing posture to the normal posture (upright posture) (S 30).

Specifically, the body mounting type power assist device 10 determines whether or not the wearer 20 has returned to the normal posture based on the (Expression 3).

$$\text{Back angular velocity} < \text{-}10 \text{ [dps]} \ldots \text{(Expression 3)}$$

The "back angular velocity" indicates the speed (degree per second) at which the back portion of the wearer 20 returns from the forward bending state, and is the angular velocity detected by the sensor 111 provided on the back portion.

The body-mounted power assist device 10 further determines whether the wearer 20 has returned from the bowing posture to the normal posture (S 40).

Specifically, when the (Expression 3) is not satisfied (NO in S 30), the body-mounted power assist device 10 further uses the (Expression 4) to determine whether or not the wearer 20 has returned from the bowing posture to the normal posture (S 40).

More specifically, the body-mounted power assist device 10 determines whether or not the wearer 20 has returned to the normal posture based on the (Expression 4).

$$\text{Left thigh acceleration} < 15 \text{ [G]} \text{ or Right thigh acceleration} < 15 \text{ [G]} \text{ or } | \text{ Left thigh acceleration - Right thigh acceleration } | < 15 \text{ [G]} \text{ or Back acceleration} < 15 \text{ [G]} \ldots \text{(Expression 4)}$$

On the other hand, when (Expression 2) is satisfied (YES in S 20), the body mounting type power assist device 10 determines whether or not the wearer 20 has stopped the forward bending operation (S 50).

Specifically, the body mounting type power assist device 10 determines whether or not the wearer 20 has stopped the forward bending operation based on the (Expression 5).

$$| \text{ Back angular velocity } | < 5 \text{ [dps]} \ldots \text{(Expression 5)}$$

When (Expression 5) is satisfied (YES in S 50), the body mounting type power assist device 10 determines whether or not the wearer 20 has started the upper body raising operation (S 60). Thus, it is determined that the wearer 20 has a heavy object such as a baggage.

Specifically, the body mounting type power assist device 10 determines whether or not the wearer 20 has stopped the upper body raising operation based on the (Expression 6).

$$\text{Back angular velocity} < \text{-}10 \text{ [dps]} \ldots \text{(Expression 6)}$$

When (Expression 6) is satisfied, the body mounting type power assist device 10 sets the rubber actuator 140 in a pressurized state. Specifically, the rubber actuator 140 is pressurized to about 0.5 Mpa.

Further, when (Expression 6) is satisfied (YES in S 60), the body mounting type power assist device 10 determines, based on the (Expression 7), whether or not the wearer 20 has stopped the upper body raising operation (S 70).

Specifically, the body mounting type power assist device 10 determines whether or not the wearer 20 has stopped the upper body raising operation based on the (Expression 7).

$$\text{Back Angle} < 5 \text{ [angle]} \text{ and Thigh Left Angle} < 5 \text{ [angle]} \text{ and Thigh Right Angle} < 5 \text{ [angle]} \ldots \text{(Expression 7)}$$

If (Expression 7) is satisfied (YES in S 70), the body mounting type power assist device 10 further determines, based on the (Expression 8), whether or not the wearer 20 has stopped the upper body raising operation (S 80).

$$\text{Left thigh acceleration} < 15 \text{ [G] and right thigh acceleration}$$
$$< 15 \text{ [G] and back angle} < 5 \text{ [angle]} \dots \text{(Expression 8)}$$

When (Expression 8) is satisfied (YES in S 70), the body mounting type power assist device 10 depressurizes the rubber actuator 140 to pressurization state (S 90).

Specifically, the body mounting type power assist device 10 depressurizes the rubber actuator 140 to pressurization based on the (Expression 9).

$$\text{Air Pressure} < \text{Pressurization state} \dots \text{(Expression 9)}$$

The "air pressure" means the pressure inside the rubber actuator 140. In step 90, the pressure is reduced to a pressurized state (about 0.04 MPa).

(5)Function and effects

According to the embodiment described above, the following effects can be obtained. Specifically, the body mounting type power assist device 10 detects that the human body 21 of the wearer 20 is in a predetermined posture, specifically, the wearer 20 is in a bent posture for lifting a heavy object. Further, when detecting that the human body 21 is in the predetermined posture, the body mounting type power assist device 10 operates the rubber actuator 140.

Thus, the lifting operation of the heavy object of the wearer 20 is assisted. In the body mounting type power assist device 10, since the power assist is performed based on the fact that the human body 21 is in the predetermined posture, the assist can be started at natural timing when the wearer 20 tries to lift the heavy object.

Further, unlike the conventional power assist suit described above, since it is not necessary to rely on the voice of the wearer 20, it can be used without any problem even in an environment with a high noise level such as in a factory or a construction site. In addition, assist can be initiated at an appropriate timing when the wearer 20 is about to lift the weight, i.e., when the wearer 20 yet has little load on the human body 21. Therefore, the wearer 20 is not burdened with lifting a heavy object until the power assist is started.

That is, according to the body mounting type power assist device 10, power assist can be started at a more appropriate timing.

In this embodiment, the timing at which the human body 21 of the wearer 20 changes from the bent posture to the rising posture is detected as a predetermined posture. Thus, the power assist can be started at an appropriate timing when the wearer 20 tries to lift the heavy object.

In the present embodiment, the body mounting type power assist device 10 includes sensors 111 provided on the thigh portion and the back portion of the human body 21. Therefore, it can be surely detected that the human body 21 changes from the bent posture to the rising posture. Thus, the wearer 20 can accurately detect the lifting operation of the heavy object and start power assist.

In this embodiment, the body mounting type power assist device 10 can detect that the human body 21 is in a predetermined posture based on the posture angle $\theta$ between the thigh portion and the back portion of the human body. Thus, the wearer 20 can more accurately detect the lifting operation of the heavy object and start power assist.

The body mounting type power assist device 10 can calculate an posture angle $\theta$ based on the detected acceleration. Therefore, the posture angle $\theta$ can be easily calculated by using a general-purpose three-axis acceleration sensor.

In this embodiment, the rubber actuator 140 (fluid pressure actuator) having the cylindrical sleeve 212 that expands and contracts with the pressure of a fluid (Air) is used.

The rubber actuator 140 follows the fine movement of the human body 21 without stress even when the body mounting type power assist device 10 is mounted on the human body 21 because the actuator body portion 210 itself expands and contracts. Therefore, more natural power assist can be realized. Power assist using a conventional motor and drive mechanism (gears, etc.) is difficult to follow such fine movements of the human body 21 without stress, and tends to cause awkward movements.

(6)Other Embodiments

Although the contents of the present invention have been described above with reference to the examples, it will be obvious to those skilled in the art that the present invention is not limited to these descriptions and that various modifications and improvements are possible.

For example, in the above-described embodiment, the step of excluding the bowing of the wearer 20 (S 20 in FIG. 5) is included, but in this step, the half-sitting state of the wearer 20 may also be excluded. In this case, for example, the half-sitting state of the wearer 20 may be eliminated based on (Expression 10).

$$\text{Air pressure} = \text{Initial} * 1.2 \text{ and } | \text{ Back angular velocity } | < 5 \text{ [dps]} \ldots \text{(Expression 10)}$$

In addition, although the operation flow of FIG. 5 shows an assist operation for lifting a heavy object, the body mounting type power assist device 10 can also assist an operation for holding the posture in a half-sitting position, as described above.

In the embodiment described above, the sensor 111 is an acceleration sensor, but a potentiometer and an encoder may be used. Alternatively, a rubber actuator 140 for sensing may be provided to detect a predetermined posture of the wearer 20 based on a size change in at least one of the radial direction $D_R$ and the axial direction $D_{AX}$ of the rubber actuator 140.

While embodiments of the invention have been described as above, it should not be understood that the statements and drawings which form part of this disclosure are intended to limit the invention. Various alternative embodiments, examples and operating techniques will become apparent to those skilled in the art from this disclosure.

[Reference Signs List]

| | |
|---|---|
| 10 | Body mounting type power assist device |
| 11 | Shoulder belt |
| 12 | Support belt |
| 13 | Waist belt |
| 20 | Wearer |
| 21 | Human body |
| 110 | Posture detection unit |
| 111 | Sensor |
| 120 | Control unit |
| 130 | Air compressor unit |
| 140 | Rubber actuator |
| 210 | Actuator body portion |
| 211 | Tube |
| 212 | Sleeve |
| 220 | Connection portion |
| 230 | Fitting |

**Claims**

1. A body mounting type power assist device mounted to a human body and including an actuator assisting a predetermined operation of the human body, comprising:

   a posture detection unit for detecting that the human body has reached a predetermined posture; and
   a control unit for actuating the actuator when the posture detection unit detects that the human body has reached the predetermined posture.

2. The body mounting type power assist device according to claim 1, wherein the posture detection unit detects a timing at which the human body changes from the bent posture to the rising posture as the predetermined posture.

3. The body mounting type power assist device according to claim 1 or 2, wherein the posture detection unit includes sensors provided on a thigh portion and a back portion of the human body.

4. The body mounting type power assist device according to claim 3, wherein the posture detection unit detects that the human body is in the predetermined posture based on a posture angle formed by the thigh portion and the back portion in a side view of the human body.

5. The body mounting type power assist device according to claim 4, wherein

   the sensor detects acceleration, and
   the posture detection unit calculates the posture angle based on the acceleration.

6. The body mounting type power assist device according to any one of claims 1 to 5, wherein the actuator is a fluid pressure actuator having a cylindrical tube that expands and contracts with the pressure of a fluid.

# FIG. 1

# FIG. 2A

FIG. 2B

# FIG. 3

# FIG. 4

EP 3 881 985 A1

FIG. 5

```
              ┌─────────┐
              │  START  │
              └────┬────┘
                   │              S10
                   ▼
         ╱─────────────────╲   YES
        ╱       START        ╲──────────────┐
        ╲ OF FORWARD BENDING  ╱              │
         ╲   *EXPRESSION 1   ╱               │
          ╲────────┬────────╱                │
                NO │                         │
                   │           S20           ▼
                   ▼    ╱──────────────────╲ YES
                  ╱    ELIMINATION          ╲────────┐
                 ╱ OF THE BOW OF WEARER      ╲        │
                 ╲    *EXPRESSION 2          ╱        │
                  ╲──────────┬──────────────╱         │
                           NO│                        │  S50
                   ┌─────────┘                        ▼
               S30 │                       ╱───────────────╲  YES
                   ▼                      ╱      STOP        ╲──────┐
         ╱─────────────────╲ YES        ╱ OF FORWARD BENDING ╲      │
        ╱  RETURNING OF      ╲─────┐     ╲   *EXPRESSION 5   ╱      │
        ╲  POSTURE           ╱     │      ╲────────┬────────╱       │
         ╲  *EXPRESSION 3   ╱      │             NO│                │
          ╲────────┬───────╱       │               │       S60      ▼
                NO │          S40  │               ▼   ╱───────────────────╲
                   ▼               │              ╱        START            ╲ YES: WEARER HAS A BAGGAGE
         ╱─────────────────╲ YES   │             ╱ OF UPPER BODY RAISING     ╲───────────┐
        ╱  RETURNING OF      ╲──────┤             ╲   *EXPRESSION 6          ╱            │
        ╲  POSTURE           ╱      │              ╲──────────┬─────────────╱             │
         ╲  *EXPRESSION 4   ╱       │                       NO│                           │
          ╲────────┬───────╱        │              ┌──────────┘              S70          ▼
                NO │                 │              │                ╱───────────────────╲ YES
                   │                 │              ▼               ╱        STOP          ╲────┐
                   │                 │                             ╱ OF UPPER BODY RAISING  ╲   │
                   │                 │                             ╲   *EXPRESSION 7        ╱   │
                   │                 │                              ╲──────────┬───────────╱   │
                   │                 │                                       NO│    S80         │
                   │                 │                                         ▼                │
                   │                 │                              ╱───────────────────╲ YES   │
                   │                 │                             ╱        STOP          ╲──────┤
                   │                 │                             ╲ OF UPPER BODY RAISING ╱     │
                   │                 │                             ╲   *EXPRESSION 8       ╱     │
                   │                 │                              ╲──────────┬──────────╱      │
                   │                 │                                       NO│        S90       │
                   │                 │                                         ▼                  │
                   │                 │                              ╱───────────────────╲ YES    │
                   │                 │                             ╱   DEPRESSURIZE       ╲───────┤
                   │                 │                             ╲ TO PRESSURIZATION STATE╱     │
                   │                 │                             ╲   *EXPRESSION 9       ╱      │
                   │                 │                              ╲──────────┬──────────╱       │
                   │                 │                                       NO│                   │
                   └─────────────────┴───────────────────────────────────────┴───────────────────┘
```

## INTERNATIONAL SEARCH REPORT

| International application No. |
|---|
| PCT/JP2019/044639 |

A. CLASSIFICATION OF SUBJECT MATTER
B25J 11/00(2006.01)i; B25J 13/08(2006.01)i; A61F 2/74(2006.01)i; A61B
5/107(2006.01)i; A61B 5/11(2006.01)i
FI: B25J11/00 Z; A61F2/74; B25J13/08 Z; A61B5/11 200; A61B5/107 300
According to International Patent Classification (IPC) or to both national classification and IPC

B. FIELDS SEARCHED

Minimum documentation searched (classification system followed by classification symbols)
B25J11/00; B25J13/08; A61F2/74; A61B5/107; A61B5/11

| Documentation searched other than minimum documentation to the extent that such documents are included in the fields searched | |
|---|---|
| Published examined utility model applications of Japan | 1922–1996 |
| Published unexamined utility model applications of Japan | 1971–2020 |
| Registered utility model specifications of Japan | 1996–2020 |
| Published registered utility model applications of Japan | 1994–2020 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| X | WO 2016/140363 A1 (NABTESCO CORPORATION) 09.09.2016 (2016-09-09) paragraphs [0017]-[0074], fig. 1-11 | 1-6 |
| A | JP 2013-075078 A (TOKYO UNIVERSITY OF SCIENCE) 25.04.2013 (2013-04-25) paragraphs [0023]-[0091], fig. 1-18 | 1-6 |
| A | JP 2013-052192 A (WAKAYAMA UNIV.) 21.03.2013 (2013-03-21) paragraphs [0042]-[0086], fig. 1-6 | 1-6 |
| A | JP 2016-159113 A (NABTESCO CORPORATION) 05.09.2016 (2016-09-05) paragraphs [0020]-[0085], fig. 1-11 | 1-6 |
| A | JP 2017-113851 A (UPR CORPORATION) 29.06.2017 (2017-06-29) paragraphs [0017]-[0061], fig. 1-8 | 1-6 |

☒ Further documents are listed in the continuation of Box C.    ☒ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 17 January 2020 (17.01.2020) | 28 January 2020 (28.01.2020) |

| Name and mailing address of the ISA/ | Authorized officer |
|---|---|
| Japan Patent Office 3-4-3, Kasumigaseki, Chiyoda-ku, Tokyo 100-8915, Japan | |
| | Telephone No. |

Form PCT/ISA/210 (second sheet) (January 2015)

**INTERNATIONAL SEARCH REPORT**

International application No.

PCT/JP2019/044639

C (Continuation). DOCUMENTS CONSIDERED TO BE RELEVANT

| Category* | Citation of document, with indication, where appropriate, of the relevant passages | Relevant to claim No. |
|---|---|---|
| A | WO 2018/203318 A1 (INNOPHYS CO., LTD.) 08.11.2018 (2018-11-08) paragraphs [0023]-[0057], fig. 1-19 | 1-6 |
| A | US 9956092 B1 (THEOBALD, Daniel) 01.05.2018 (2018-05-01) column 2, line 54 to column 17, line 7, fig. 1-12 | 1-6 |

Form PCT/ISA/210 (continuation of second sheet) (January 2015)

| INTERNATIONAL SEARCH REPORT<br>Information on patent family members | | International application No.<br>PCT/JP2019/044639 | |
|---|---|---|---|
| Patent Documents referred in the Report | Publication Date | Patent Family | Publication Date |
| WO 2016/140363 A1 | 09 Sep. 2016 | JP 2016-159109 A | |
| JP 2013-075078 A | 25 Apr. 2013 | (Family: none) | |
| JP 2013-052192 A | 21 Mar. 2013 | (Family: none) | |
| JP 2016-159113 A | 05 Sep. 2016 | US 2019/0029910 A1 paragraphs [0057]-[0122], fig. 1-11 WO 2016/140361 A1 EP 3266422 A1 CN 107530175 A | |
| JP 2017-113851 A | 29 Jun. 2017 | (Family: none) | |
| WO 2018/203318 A1 | 08 Nov. 2018 | CA 3062117 A1 paragraphs [0023]-[0057], fig. 1-19 | |
| US 9956092 B1 | 01 May 2018 | US 10028878 B1 | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2011251057 A
- JP 2015047662 A

- WO 2017010304 A